# EUROPEAN PATENT APPLICATION

(11) **EP 1 091 003 A2**
(43) Date of publication of application: **11.04.2001**
(21) Application number: 00308502.4
(22) Date of filing: 28.09.2000
(51) Int. Cl.: C12Q 1/68

(54) **Improvements in or relating to detection of microorganisms in plant material**

(30) Priority: 30.09.1999 GB 9923022
(71) Applicant: The Minister of Agriculture, Fisheries & Food in Her Britannic Majesty's Government of the United Kingdom and Northern Ireland, London SW1P 3JR (GB)
(72) Inventor: Taylor, Emily Jasmine Alice, Cambridge CB4 3LF (GB)
(74) Representative: Matthews, Heather Clare

(57) **Abstract**

PCR primers are provided that can enable detection of *Pyrenophora graminea* in plant material, particularly barley seeds, enabling discrimination between *P. graminea* and other *Pyrenophora* species.

## Description

### Field of the Invention

This invention relates to detection of microorganisms in plant material, eg plant seeds, plant organs, plant leaves, stem tissue, plant debris etc, and concerns particularly, but not exclusively, detection of *Pyrenophora graminea* in seeds from cereal crops, particularly barley.

### Background to the Invention

Barley diseases caused by *Pyrenophora* species result in severe economic loss for growers in many different countries (Stevens *et al,* 1998 and Stevens *et al*, 1997). Three distinct types of disease are found: leaf stripe symptoms, caused by *P. graminea*; net blotch, caused by *P. teres* f. sp. *teres*; and the spot form of net blotch, caused by *P. teres* f. sp. *maculata*. These species have different forms of disease transmission. *P. graminea* is transmitted entirely by seed and cannot cause disease by direct leaf infection, whereas local transmission from plant debris is thus more significant for *P. teres* species. Unambiguous identification of the pathogens is thus necessary for development of appropriate control strategies. The most effective method of controlling seedborne diseases is through seed health certification. Although *P. graminea* is not directly covered by existing seed certification procedures there are voluntary standards which have been adopted (Cockerell *et al*., 1995).

Organomercury fungicides were used to control these diseases until their use was banned in 1992. With increasingly tight regulation of pesticide usage, it seems probable that in the long term the most effective means of seedborne disease control will be through seed health certification and the introduction of new resistant barley cultivars.

In current seed health tests, the morphological characteristics of the pathogens are observed after incubating barley seed on agar for a week (Rennie & Tomlin, 1984). However, these techniques are both time-consuming and labour-intensive and fungal identities can sometimes only be confirmed after prolonged pathogenicity testing.

Seed processors operate under critical time constraints during the period between harvest and delivery of seed, and current seed health testing techniques do not allow sufficient time for the testing of large number of seed lots. Fungicide treatment has thus become the only acceptable guarantee against the development of damaging levels of seed-borne disease, and this is estimated to cost UK barley growers around £10 million per year (Cockerell & Rennie, 1996).

The present invention is based on work leading to the development of primers specific for *P. graminea* for use in the polymerase chain reaction (PCR).

### Summary of the Invention

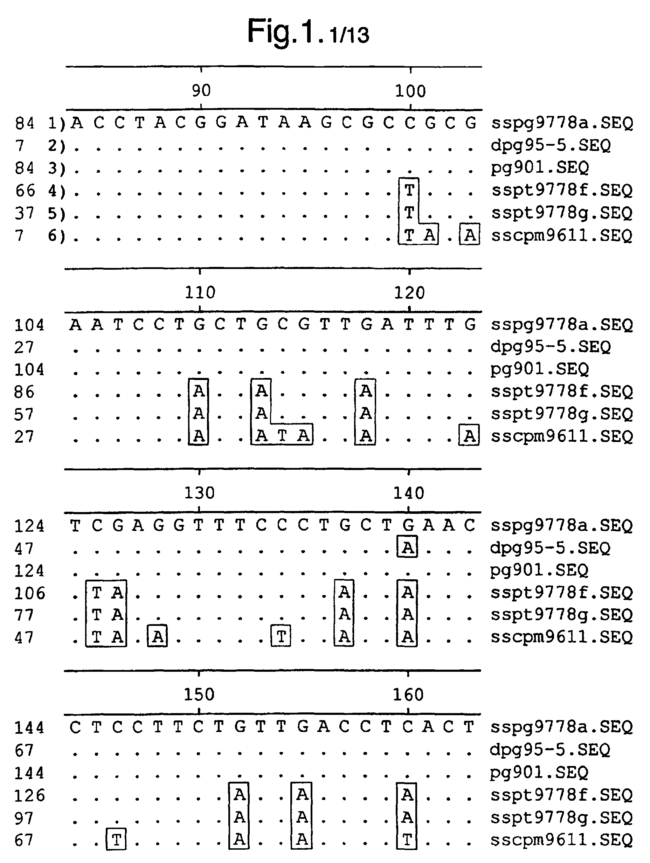

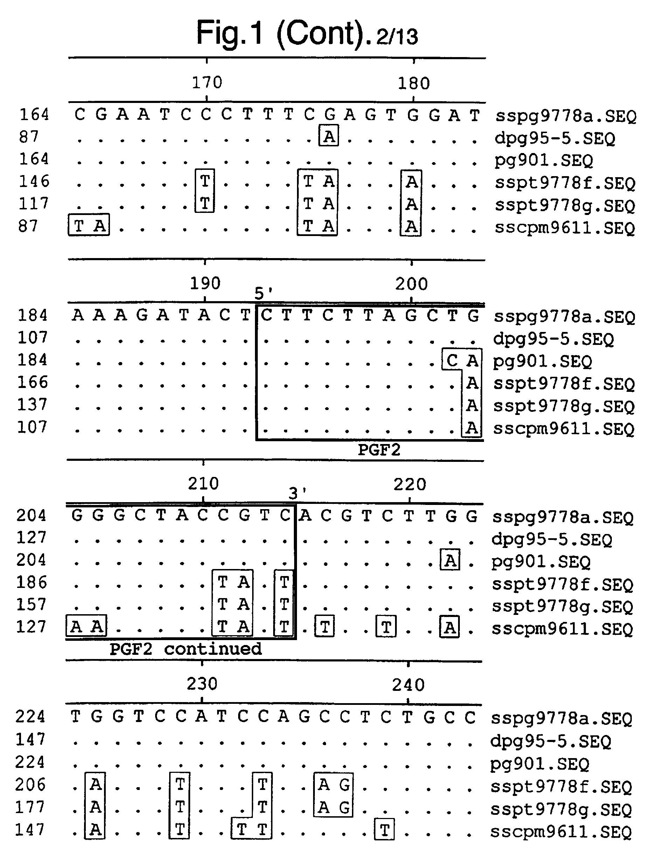

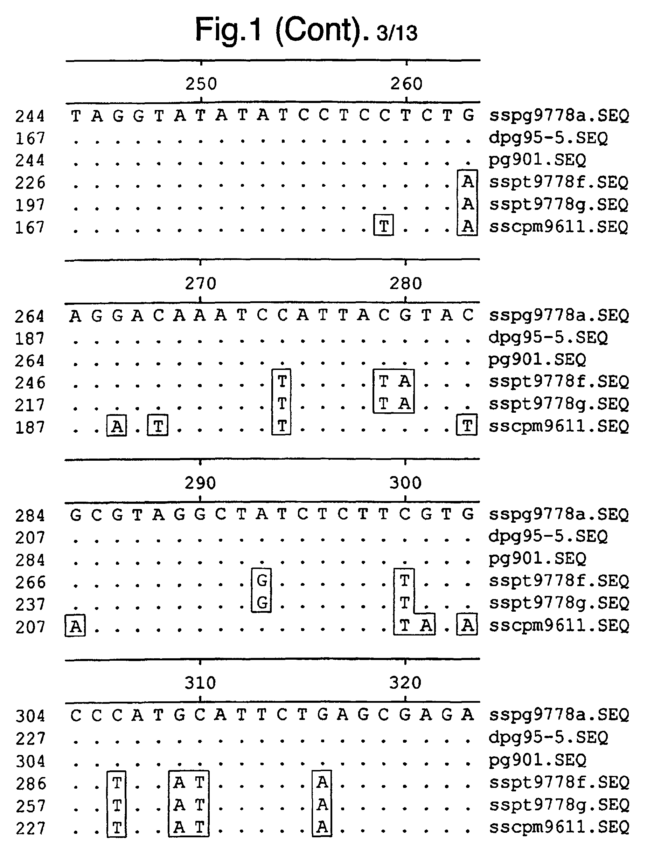

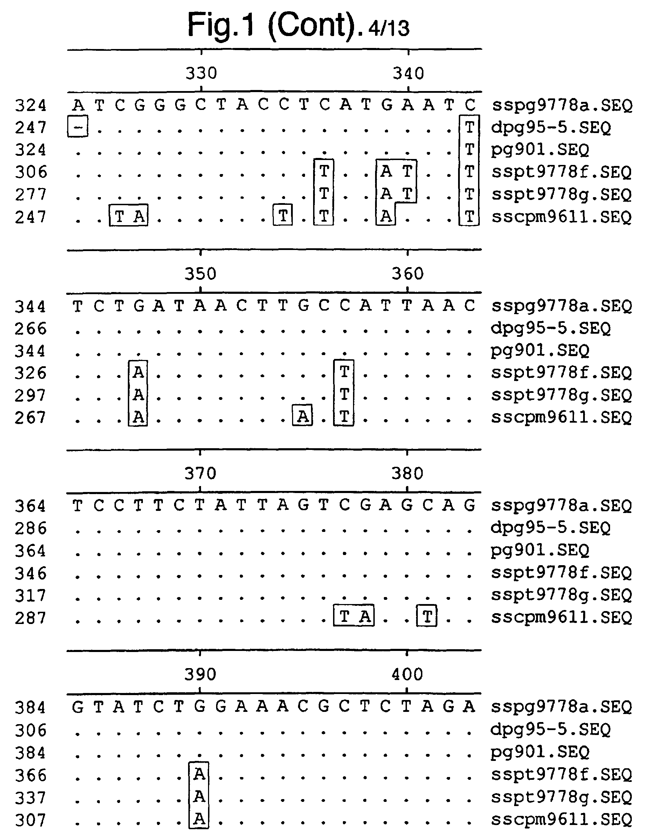

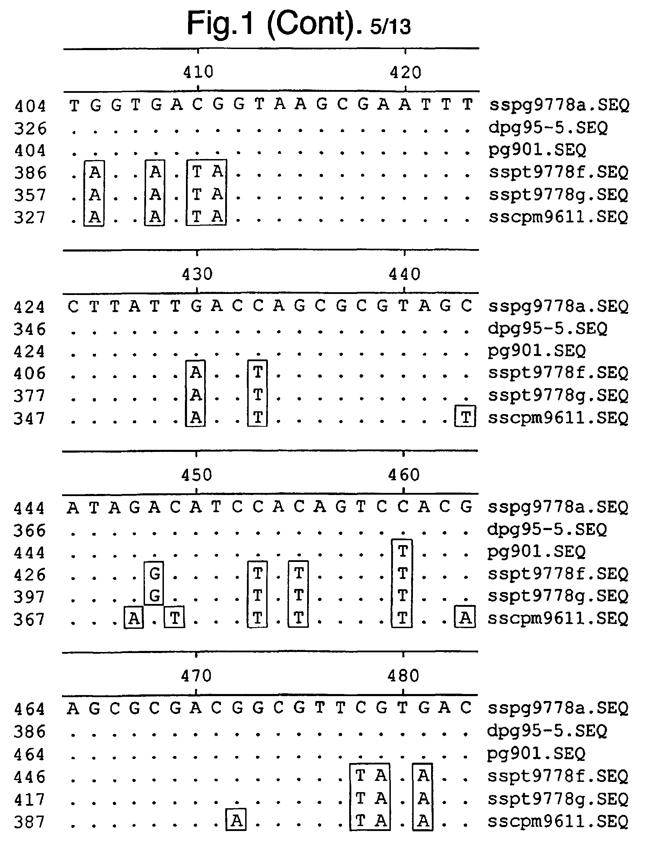

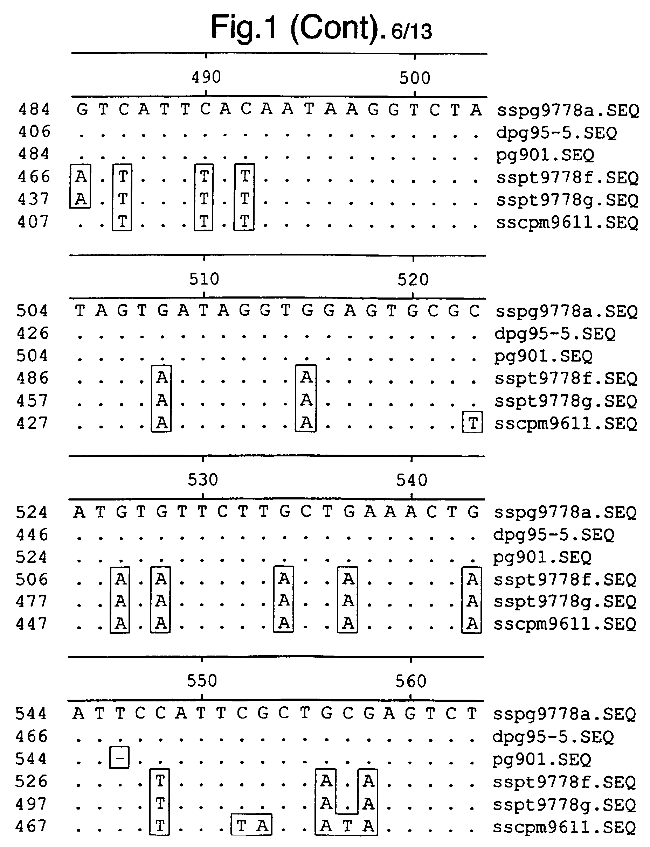

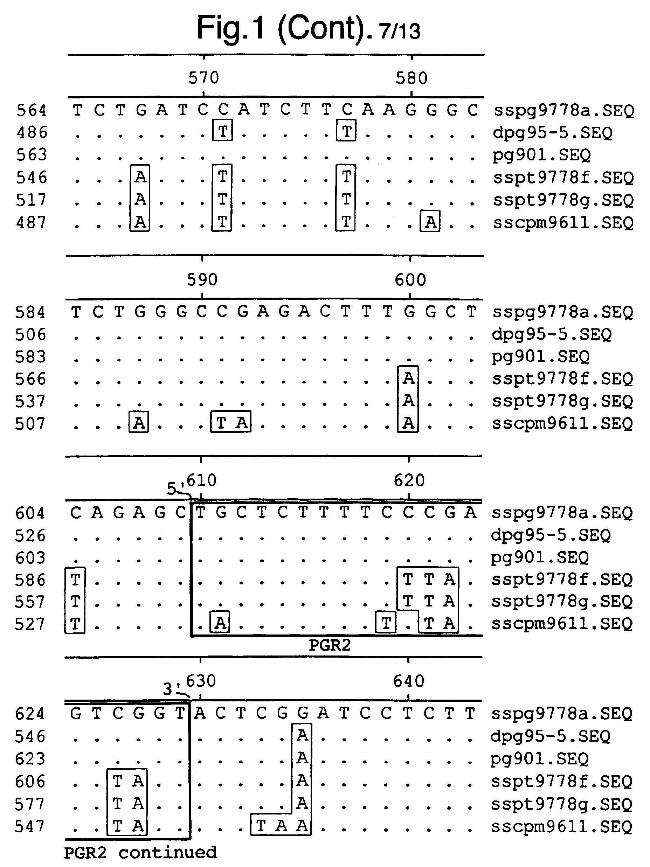

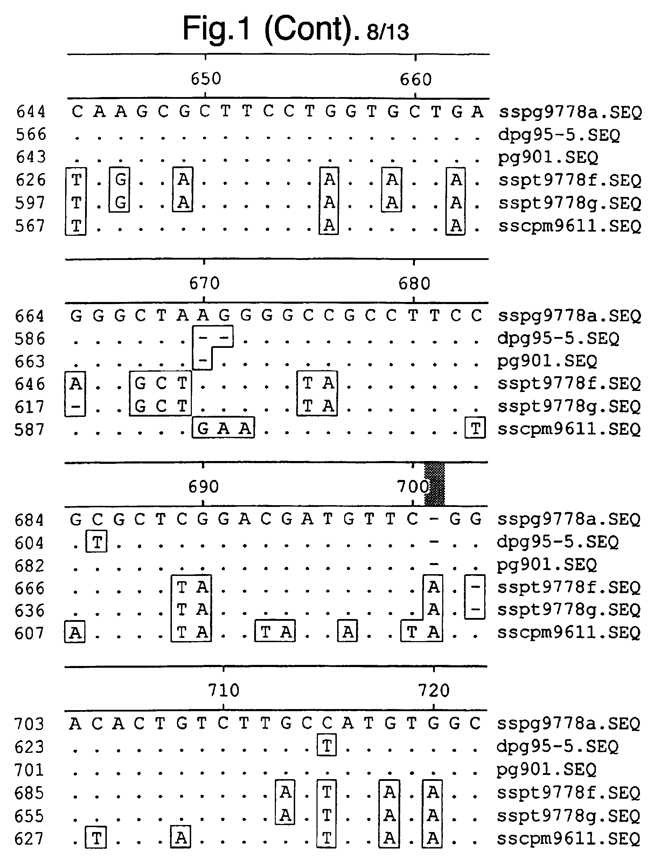

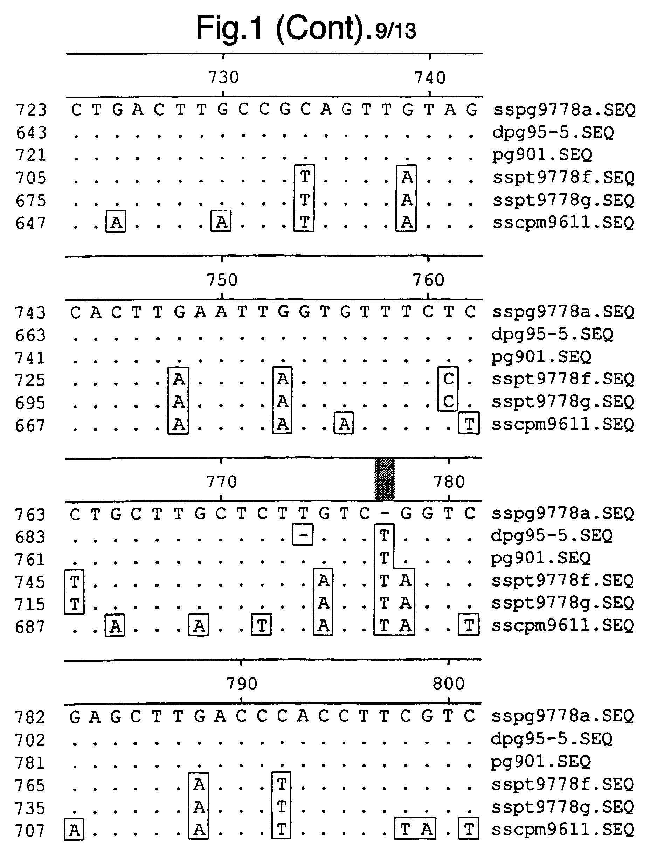

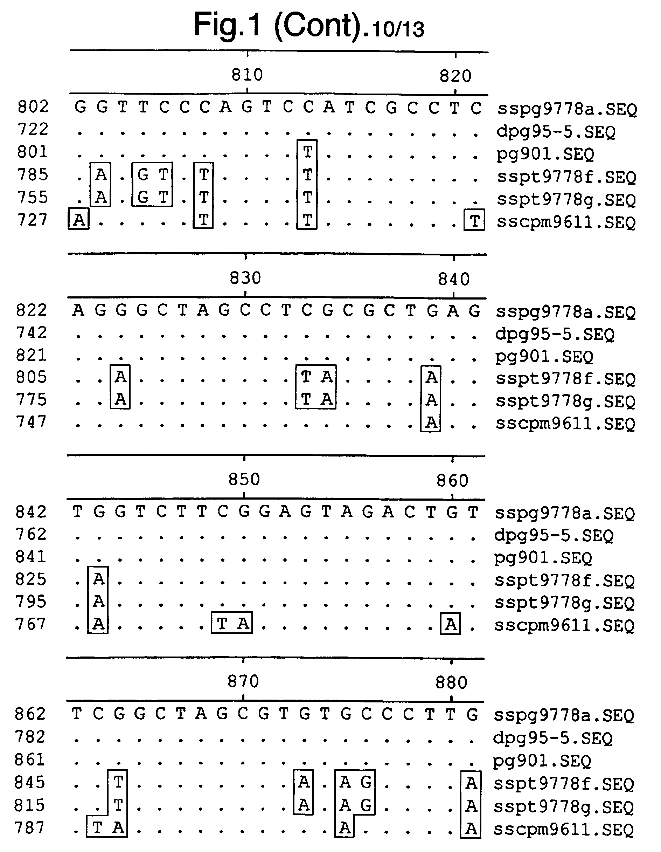

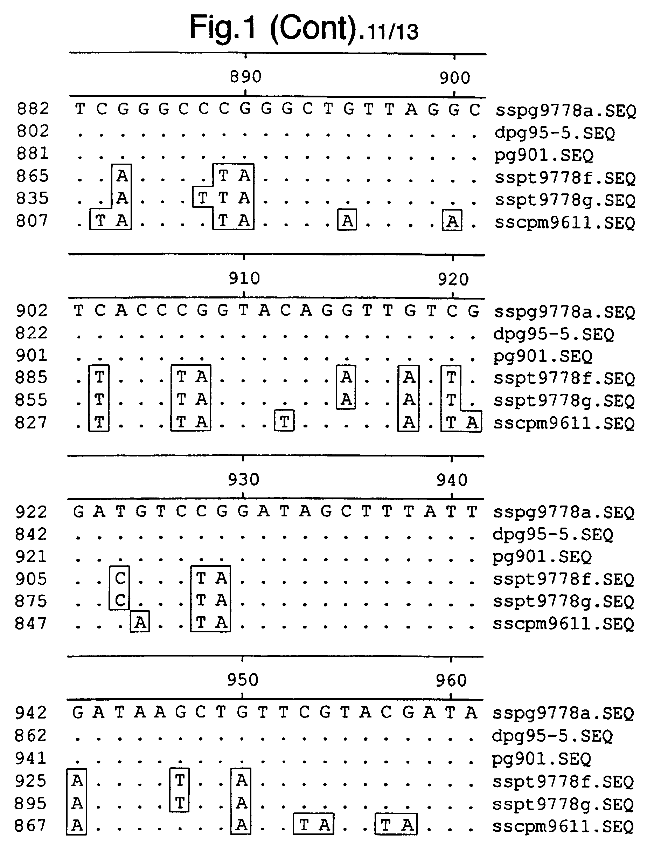

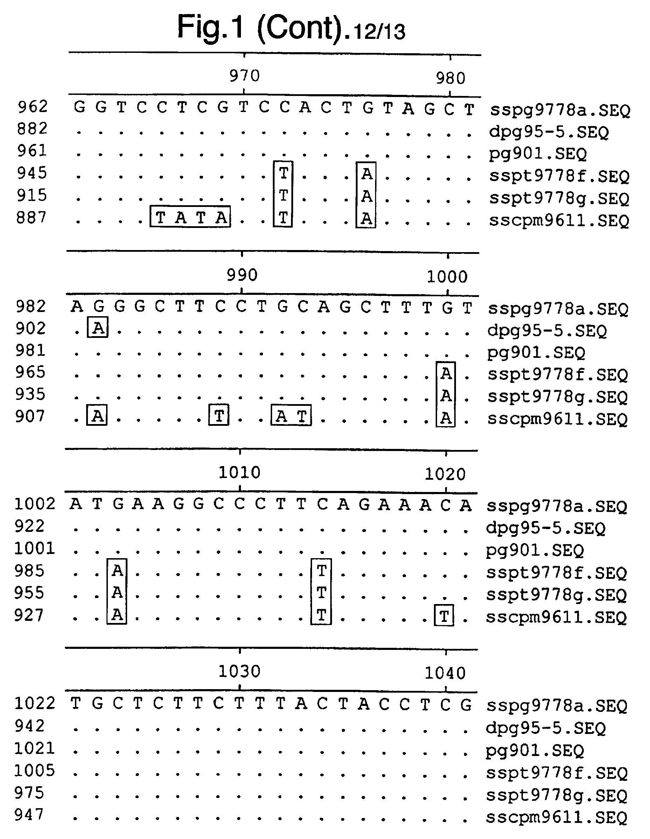

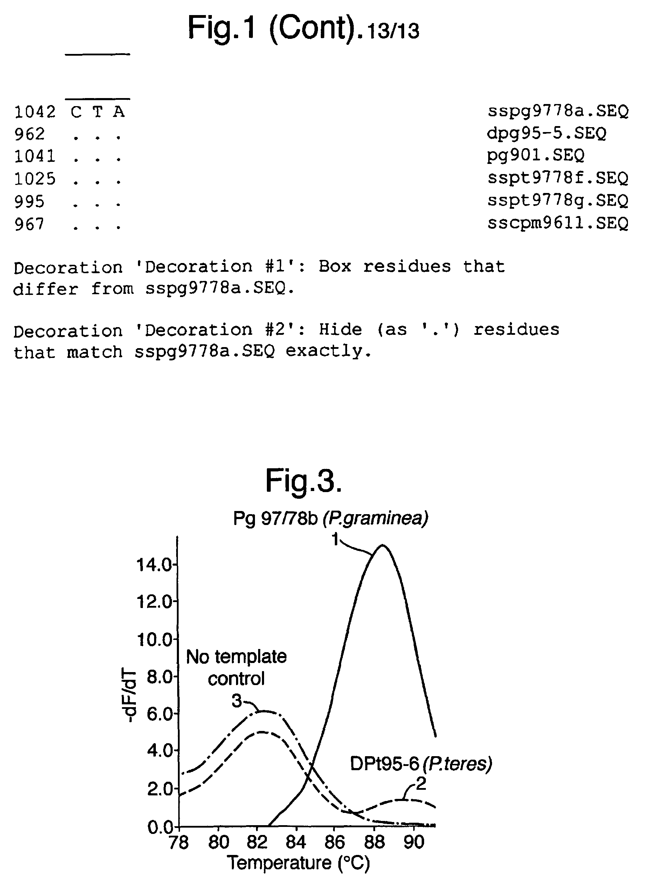

In a first aspect the invention provides a nucleotide sequence obtainable from *Pyrenophora graminea* comprising the sequence presented as SEQ ID No: 1 in Figure 1 and Table 4 or a variant of the sequence.

The term variant is intended, inter alia, to cover sequences which will hybridise to SEQ ID No. 1 or the complement thereof under stringent hybridisation conditions, eg as described by Sambrook *et al*, ie washing with 0.1 xSSC, 0.5% SDS at 68°C. Variants may, for example, comprise small (e.g. up to 10-20 base) insertions, deletions or substitutions relative to SEQ ID No: 1.

The invention in particular covers SEQ ID No: 1 and the 5 variants thereof shown in Figure 1. Preferably variants of SEQ ID No: 1 are also sequences obtainable from *P. graminea*, such as the sequences shown as lines 2 and 3 of Figure 1.

This sequence information has been used to design oligonucleotide primers useful in PCR to identify particular *Pyrenophora* species and to distinguish between different *Pyrenophora* species, in particular to identify *P. graminea* and distinguish *P. graminea* from other *Pyrenophora* species, particularly *P. teres*. Other examples include primers able selectively to identify, eg, *P. teres* f. sp. *teres* or *P.teres* f. sp. *maculata.*

In a further aspect the invention provides an oligonucleotide comprising at least 10 consecutive bases as occurring in a nucleotide sequence in accordance with the first aspect of the invention, preferably SEQ ID No: 1, or the complement of such a sequence.

The oligonucleotide preferably comprises at least 15, more preferably about 20, consecutive bases.

The oligonucleotide desirably comprises a sequence which will hybridise under stringent PCR conditions (eg 30 cycles of 1 minute at 92°C, 1 minute at 50°C, then 1 minute at 72°C, with reaction completed after a final extension step at 72°C for 10 minutes) to a nucleotide sequence present in the genome of only one *Pyrenophora* species, eg which will hybridise to a sequence present in the genome of *P. graminea* but will not hybridise to sequences present in other *Pyrenophora* species, particularly *P. teres*.

Another aspect of the invention provides a pair of oligonucleotides, one comprising at least 10 consecutive bases as occurring in a first portion of a nucleotide sequence in accordance with the first aspect of the invention, preferably SEQ ID No: 1, and the other comprising at least 10 bases complementary to a second portion of a nucleotide sequence in accordance with the first aspect of the invention, preferably SEQ ID No: 1, such that the oligonucleotides are capable of acting as primers in a polymerase chain reaction and will amplify a region of the sequence of only one *Pyrenophora* species, particularly *P. graminea.*

The oligonucleotides of such a pair preferably each comprise at least 15 bases, more preferably about 20 bases.

The invention also covers an oligonucleotide having the sequence 5'CTTCTTAGCTGGGCTACCGTC 3' (SEQ ID No: 2). This sequence is also referred to as PGF2.

The invention further covers an oligonucleotide having the sequence 5' ACCGACTCGGGAAAAGAGCA 3' (SEQ ID No: 3). This sequence is also referred to as PGR2.

Another aspect of the invention resides in a pair of oligonucleotides, comprising PGF2 and PGR2.

This invention also covers an oligonucleotide having the sequence 5' GTTTCTACCTACGGATAGC 3' (SEQ ID No: 4). This sequence is also referred to as PGF1.

The invention further covers an oligonucleotide having the sequence 5'TAGCGAGGTAGTAAAGAAGC 3' (SEQ ID No: 5). This sequence is also referred to as PGR1.

A further aspect of the invention comprises a pair of oligonucleotides, comprising PGF1 and PGR1.

Oligonucleotides in accordance with the invention can be used as PCR primers capable of selectively amplifying DNA from, say, *P. graminea*, eg present in DNA extracted from plant material, particularly barley seeds, and so indicating the presence of this particular plant pathogen. Particularly when using PGF2 and PGR2 as primers, complete discrimination between *P. graminea* and other *Pyrenophora* species and also other fungal species commonly isolated from barley seed has been possible.

In a further aspect the invention provides a method of detecting the presence of a specific *Pyrenophora* species, particularly *P. graminea*, in plant material, particularly barley seeds, comprising
a) extracting nucleic acid from a sample of the plant material;
b) performing PCR using as primers a pair of oligonucleotides in accordance with the invention; and
c) detecting the presence of any PCR products.

*P. graminea* infection is located in the pericap of the seed (Platenkamp, 1976), and is not deep-seated, so extraction and isolation of fungal DNA can be performed relatively simply and quickly. *P. graminea* is conveniently released or recovered from seeds by a technique involving softening the outer investing layer of the seed (ie the pericarp if present or the outermost tissue layer of a seed lacking a pericap) eg by soaking in an aqueous medium; and causing at least partial disruption of the outer investing layer, eg by mechanical agitation, thereby releasing *P. graminea* and other microorganisms, if present, from the seed. For further details of this approach, see Thomas *et al* 1998. Nucleic acid may then be extracted, eg in known manner, from material released or recovered from seeds in this way.

Techniques for performing PCR are well known to those skilled in the art.

It is preferred to detect PCR products using a LightCycler (Idaho Technology) fluorescent detection system. This equipment allows rapid (approximately 5 times faster than with a conventional PCR machine) real time detection, is suitable for automation and can enable the kinetics of the PCR reaction to be observed. The Light Cycler can perform PCR in less than 15 minutes, making the assay extremely rapid, thus allowing for processing of a greater number of samples than has hitherto been possible. It is, of course, also possible to perform PCR using other conventional PCR equipment instead of such rapid PCR equipment.

Using the LightCycler equipment, a PCR test can be completed in 24h, with batch sizes up to 30 for a single operator. With increasing automation, it is probable that several batches could be processed in a day by one person. Since winter barley seed in the UK has to be processed within about a two month period from harvest to sowing, the method would offer sufficient capacity for the routine testing of all seed lots, and thus the potential for treating seed according to disease risk. These has been a steady decline in the incidence and severity of *P. graminea* in seed stocks submitted for testing (Cockerell & Rennie, 1996, Thomas *et al*, 1998), though a low incidence is recorded in most years. The PCR test is not quantitative, but could be used as a very rapid primary screen to identify the few positive samples which are likely to occur. Agar plate testing could be used to determine if samples are above the threshold level of 2%, or all positive samples could be tested routinely as a precaution.

The invention also includes within its scope a kit for performing a polymerase chain reaction, comprising a pair of oligonucleotides in accordance with the invention, preferably PGF2 and PGR2.

The kit preferably further comprises one or more of the following: reaction buffer, polymerase, nucleotide triphosphates, labelling reagents and instructions for use.

The invention can thus provide a simple, rapid and effective test for the identification of specific *Pyrenophora* species, particularly *P. graminea*. A PCR based approach has the advantages of rapidity and can cope with a high-through put of seed samples at harvest, with extreme sensitivity and specificity. A new PCR seed health test will provide farmers the means to apply seed treatment according to need without the risk of delaying the processing and delivery of seed. In particular, the invention can provide a PCR seed health test to allow unambiguous identification of *P. graminea*. on barley.

Oligonucleotides in accordance with the invention can also be used in multiplex testing for different *Pyrenophora* species with a well defined melting curve of specific PCR products using Syber Green I or molecular probes with different fluorescent dyes (Woo *et al*, 1997).

Two strategies were adopted to design specific primers for use in PCR. One approach was to design specific primers based on sequence information from the Internal Transcribed Spacer (ITS) regions of ribosomal genes. This has been successfully done in other organisms to develop specific diagnostic tests (e.g. Xue *et al*., 1992). Sequence information for the ITS regions was obtained by PCR using the universal primers designed for this purpose (White *et al*., 1990). The results indicated that there was a higher degree of intraspecific variation than inter-specific sequence difference between the three *Pyrenophora* species which meant that it was not possible to design robust species specific primers (Stevens *et al,* 1998 and Stevens *et al,* 1997). This sequence information supports other research (Smedegaard-Peterson, 1983) which indicates that the three *Pyrenophora* species are not separate species.

Another approach used to distinguish *P. graminea* was based on random PCR amplification (RAPD) arbitrary primers, which has proven to be a powerful tool for genetic analysis elsewhere, particularly as no prior sequence information is required (Welsh & McClelland, 1990). Preliminary work by Reeves & Ball (1991) suggested the potential value of RAPD's in differentiation of the *Pyrenophora* species. pathogenic on barley.

RAPDs has been used successively as a technique for identifying diagnostic markers in fungi (Parry and Nicholson, 1996, Johanson *et al*, 1994). Obtaining a suitable marker that could identify *P. graminea* and differentiate it from *P. teres* and *P. teres* f. sp. *maculata* was achieved but involved a more complex process that what has been observed by other researchers (Parry and Nicholson, 1996, Johanson *et al*, 1994). This was because the selected RAPD product shown from a hybridisation experiment not to be species specific. Primers PGF2/PGR2 had therefore to be designed from the partially sequenced RAPD product of not only isolates of *P. graminea* but also *P. teres* and *P. teres* f. sp. *maculata* isolates to fined base pair differences to achieve species specificity.

The invention will be further described, by way of illustration, with reference to the accompanying drawings, in which:
Figure 1 is an alignment report of 14699 meg, using Clustal method with weighted residue weight table, with sequences for 3 different isolates of *P. graminea,* 2 different isolates of *P. teres* f.sp. *teres* and 1 isolate of *P. teres* f.sp. *maculata*, with bases not matching the top sequence highlighted by a box. The 6 sequences, with EMBL Nucleotide Sequence Database accession numbers, are as follows:
   1. *P. graminea* isolate code SS pg 97-98a (UK) (AJ 243140) (SEQ ID No: 1) (also shown in Table 4).
   2. *P. graminea* isolate code dpg 95-5 (Demark) (AJ 243050).
   3. *P. graminea* isolate code pg 901 (UK) (AJ 243049).
   4. *P. teres* f. sp. *teres* isolate code sspt 97-78f (UK) (AJ 243141).
   5. *P. teres* f. sp. *teres* isolate code sspt 97-78g (UK) (AJ 243142).
   6. *P. teres* f. sp. *maculata* sscpm 96-11 (Canada) (AJ 243143).
   The sequence of primer PGF2 is marked, and the sequence to which primer PGR2 is complementary is also marked on Figure 1.

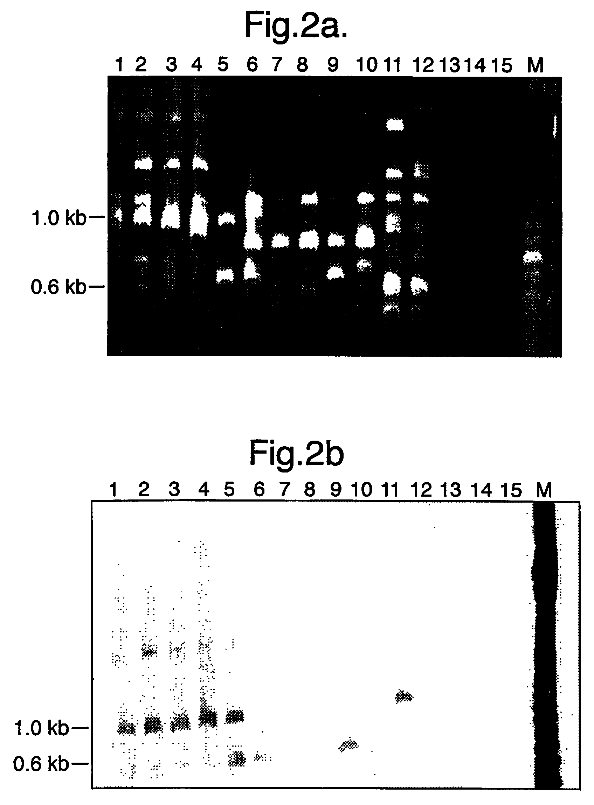

Figure 2a is a RAPD profile of 14 isolates of *Pyrenophora* species produced following amplification with primer OPT 16.
Figure 2b is an autoradiograph of the RAPD profile of Figure 2a hybridised with probe P1. In Figures 2a and 2b, the lanes are as follows:
   Lanes 1-5; *P. graminea* (Pg 95-3, Pg 95-7, Pg 91-19, Pg 90-38 and Pg 95-17), lanes 6-10 and 14; *P. teres* (Pt 93-14, Pt 93-9, Pt 93-18, Pt 93-20 and Pt 93-22), lanes 11-12; *P. teres* f. sp. *maculata* (DPt 95-6 and DPt 95-3), lane 13; *Basidiomycete* sp 95-5, lane 14; negative control (no template) and lane 15; 100 bp DNA marker.

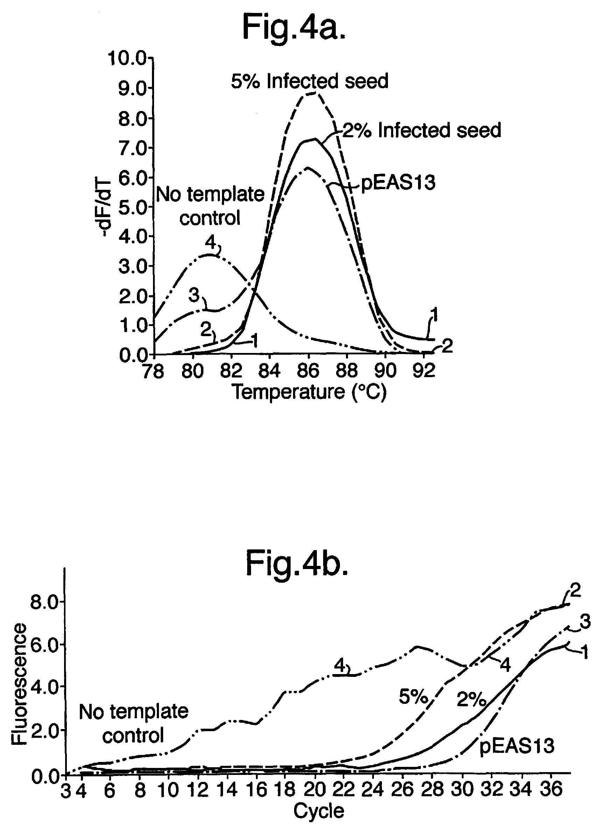

Figure 3 is a graph of -dF/dT versus temperature (°C) showing melting peaks of specific PCR product from representative strain (Pg 97/78b) of *P. graminea* (curve 1), non-specific product and product dimer from representative strain (DPt 95-6) of *P. teres* (curve 2) and no template control (curve 3).
Figure 4a is a graph of -dF/dT versus temperature (°C) showing melting peaks of specific PCR product from DNA extracted directly from 2% and 5 % *P. graminea* infected barley seed (curves 1 and 2) and from positive control of plasmid pEAS13 (curve 3) which contains *P. graminea* DNA insert. No template control (curve 4) has a melting peak from primer dimer formation.
Figure 4b is a graph of fluorescence versus cycle number for continuous monitoring of fluorescence emission during rapid cycle PCR of template DNA from 2% and 5% *P. graminea* infected seed (curves 1 and 2), from positive control of 12pg plasmid pEAS13 (curve 3) which contains *P. graminea* DNA insert and no template control (curve 4).

### Materials and methods

### Collection of fungal isolates

The *Pyrenophora* spp. isolates used in this work were obtained from geographically diverse areas, as shown in Table 1. Fungal isolates derived from single conidia were identified, cultivated and maintained as previously described by Stevens *et al,* (1998), and Stevens *et al,* (1997).

### Collection of barley seed samples

Barley seed samples which had tested positive for *P. graminea* infection by agar testing (Rennie & Tomlin,1984) were obtained from the Official Seed Testing Stations in England (particularly National Institute of Agricultural Botany, Huntingdon Road, Cambridge, CB3 0LE, UK (NIAB)) and from UniRoyal Chemical Ltd. Lots with varying target levels of infection between 1 and 15% were constructed by mixing different proportions of an infected and non-infected seed lot (Table 2) using the thousand grain weights of each lot to determine the amounts to be used. Twenty replicate samples of 200 seeds for each infection level were prepared by blending and dividing the mixed bulk with a Garnet and Pascall centrifugal sample divider. Ten replicates were assessed using an agar plate test, and ten with the PCR assay.

### Fungal DNA extraction

Genomic DNA was extracted from single spore derived fungal cultures as described by Stevens *et al*., 1996, with the modification of incubating the fungal culture in potato dextrose broth for 4 days. The DNA samples were then further purified with a commercial resin and silica membrane column (Wizard™ DNA clean-up system, Qiagen) according to the manufacturer's instructions.

### DNA extraction from P. graminea infected barley seed.

Two hundred infected seeds were soaked in 20 ml of TE buffer (10 mM Tris-HCl, 1 mM EDTA pH 8.0) for 5 minutes in a plastic stomacher bag (A.J.Seward, Suffolk, UK) with the dimensions 177 x 304 mm. This seed soak mixture was then blended for 60 seconds in a stomacher (Lab-Blender™ 400, A.J.Seward, Suffolk, UK). The seed soak liquor was centrifuged tube at 4000g, 4°C for 10 minutes. The supernatant was removed and the pellet freeze-dried. The freeze-dried pellet containing microorganisms extracted from the seeds was ground to a powder in the presence of liquid nitrogen. DNA was extracted from the powder using a silica membrane spin cup kit (NucleoSpin™ plant kit, Machery-Nagel GmbH & Co, Duren, Germany) as described by manufacturer's instructions for the plant procedure, with three minor modifications; the addition of two washes with buffer CW to remove contaminants that might inhibit the PCR, and a longer centrifugation step of 2 min. at 11500g to ensure complete removal of buffer C5.

### RAPD assay

Amplification conditions were carried out as described by Stevens *et al*., 1996. RAPD-PCR was performed with 60 primers (Genosys Biotechnologies, Cambridge, UK) 10 nucleotides in length to produce RAPD's from isolates of *Pyrenophora* spp. pathogenic on barley.

### Southern blotting and hybridisation

RAPD products were size fractionated on a 1.4% agarose gel and transferred to a nylon membrane as described by Sambrook *et al*, (1989). A DNA probe was made by cutting a selected 1 kb DNA band directly from an agarose gel and eluting the DNA with the Qiakquick™ (Qiagen, Sussex, UK) system as directed by the manufacturer. The DNA probe was non-radioactively labelled and hybridised under stringent conditions using ECL (Amersham Pharmacia Biotech, Buckinghamshire, UK), according to the instructions given by the manufacturer.

### Development of PCR assay

A 1 kb amplification product amplified with primer OPT16 (5' GGTGAACGCT 3') (SEQ ID No: 6) from *P. graminea* was isolated, cloned into pGEM-T vector (Promega, Southampton, UK) and transformed into JM109 high efficiency competent cells (Promega, Southampton, UK) according to suppliers instructions. The insert fragment ends of selected clones were sequenced using the ABI Prism Dye Terminator Cycle Sequencing Ready Reaction Kit (Perkin Elmer Applied Biosystems, California, USA) according to manufacturers instructions. Primers for specific PCR were designed using 'Primer select', from DNASTAR™ software (New York, USA) based on sequence information obtained as described above.

Two primer pairs PGF1/PGR1 (5' GTTTCTACCTACGGATAGC 3') (SEQ ID No: 4)/ (5' TAGCGAGGTAGTAAAGAAGC 3') (SEQ ID No: 5) and PGF2/PGR2 (5' CTTCTTAGCTGGGGCTACCGTC 3') (SEQ ID No: 2)/ (5' ACCGACTCGGGAAAAGAGCA 3') (SEQ ID No: 3) were generated and tested against DNA of a range of *Pyrenophora* spp. and other fungi isolated from barley seed samples.

### PCR screening of P. graminea DNA with primers PG1 F/R

DNA extracted from fungal cultures was amplified using an initial denaturing step of 92°C for 10 min. This was followed by 30 cycles of 1 min at 92°C, 1 min at 50°C and then 1 min at 72°C. The PCR was completed after a final extension step at 72°C for 10 min.

### Rapid cycle PCR with continuous monitoring of product.

DNA was extracted from fungal cultures or infected seed as described previously. A total reaction volume of 10 µl was used for the PCR. Reactions consisted of a 5 µl master-mix (dNTPs, BSA, 3mM MgCl₂, *Taq* polymerase and reaction buffer) (Biogene, Cambridgeshire, UK), 0.5 µl of 1/1000 dilution SYBR Green 1 concentrate (Biogene Cambridgeshire, UK), 0.5 µM PGF2 F/R and 2.5 µl of DNA extracted from seed or 2.5 µl of 1-10 ng/µl fungal DNA. Amplification was carried out in a LightCycler (Biogene, Cambridgeshire, UK) with an initial denaturing step at 95°C for 30 s followed by 35 cycles of 95°C for 0 s, 68°C for 0 s and 74°C for 5 s. (Where a time of 0 s is specified for a particular temperature, the equipment is programmed to reach the specified temperature but not to maintain that temperature for any significant time.) The temperature transition rate was set at 20°C per second. Acquisitions of fluorescence signal were carried out at the end of every extension step for 50 msec. For distinguishing specific products from non-specific products and primer dimers, a melting curve was obtained immediately after amplification by holding the temperature at 74°C for 5 s followed by a gradual increase in temperature to 100°C at a rate of 0.1°C/s, with the fluorescent signal acquisition mode set at step. The fluorescent data was converted into melting peaks as described by Ririe *et al*, 1997 and Woo *et al*, 1998. A graph was then plotted with the negative derivative of fluorescence with respect to temperature ( -dF/dT).

In samples that produced negative results, a repeat PCR test was carried out to check that an error had not occurred in performing the PCR reaction. In samples that produced negative results in the repeat PCR experiment, a further PCR assay was performed to check that DNA extraction from the sample had been effective and was amplifiable. This was carried out by using universal ITS primers 4 & 5 (White *et al*, 1990) that amplify fungal and barley DNA essentially as described by Stevens *et al,* 1998 and Stevens *et al*, 1997, but with a modification of the annealing temperature which was lowered to 53 °C.

### RESULTS

### RAPDs of P. graminea

RAPD analysis was performed on five isolates of *P. graminea,* five isolates of *P. teres* f. sp.*teres*, two isolates of *P. teres* f. sp. *maculata* and two isolates of *P. avenae* using 60 different 10-mer primers. Primer OPT16 produced one major amplification product (P1) 1 kb in size that was common to all the *P. graminea* isolates but not in the other *Pyrenophora* spp. tested (Figure 2a). P1 was selected for further study. None of the other 59 primers produced RAPD profiles that differentiated *P. graminea* from the other *Pyrenophora* spp. tested.

### Hybridisation of 1 kb P. graminea RAPD product

P1 was used to probe a Southern blot of RAPD products with primer OPT16 from *P.graminea* and other *Pyrenophora* spp (Figure 2b). P1 hybridised three DNA fragments for all five isolates of *P. graminea* tested. The strongest hybridisation was to the 1 kb fragment found in the *P. graminea* isolates and the region from which the probe was isolated from. This indicated that the 1 kb RAPD DNA product for all the *P. graminea* isolates observed on agarose gel shared homology. There was also hybridisation to a DNA product from two *P. teres* isolates (0.6 kb and 0.7 kb), and one *P. teres* f. sp. *maculata* isolate (1.1 kb). This result indicated that P1 was not species specific for *P. graminea* as it shared homology with RAPD products amplified from other *Pyrenophora* spp. P1 probe was chosen to be cloned (pEAS13) and selected for sequencing as although there was some cross hybridisation with *P. teres* and *P. teres* f. sp. *maculata* there may be sequence differences between them that could be used for designing primers specific for *P. graminea.*

### Development of specific PCR primers for P. graminea

Primer pair PGF1/PGR1 generated from clone pEAS13 was used to do a preliminary screen for specificity of 26 isolates (Table 1) of *P. graminea*, 14 isolates of *P. teres* and four *P. teres* f. sp. *maculata* isolates. The PCR results indicated that the primers were not fully species specific as six *P. teres* isolates and one *P. teres* f. sp. *maculata* isolate amplified with this set of primers. PCR products that were amplified with this set of primers (PGF1/PGR1) were cloned and the insert ends sequenced from three isolates of *P. graminea,* two isolates of *P. teres* and one of *P. teres* f. sp. *maculata.* Sequence data (Figure 1 and Table 4) was compiled and aligned using a combination of Seqman™ and Megalign™ computer programmes (DNASTAR, New York, USA) for all the isolates so that nucleotide differences between *P. graminea* and the other *Pyrenophora* species could be observed more easily. Primers PGF2/PGR2 were designed taking into account criteria such as product length, optimal PCR annealing temperature, and likelihood of primer self-annealing. Base mispairs between the primer and non-*P. graminea* sequences were placed near the 3' end of the primer to maximise specificity.

### Rapid cycle PCR and melting curve of P. graminea specific product.

Thirty seven isolates of *P.graminea* genomic DNA were amplified with primers PG2 F/R and produced a melting curve with a Tm of 86.5-88.5 °C (Table 1 and Figure 3), indicating that they all produced the same product. The 2°C variation (86.5-88.5°C) observed for the melting curve of the specific PCR product in different experiments may be due to slight variation in the concentration of SYBER Green I dye between batches due to pipetting error. It is known that the addition of intercalaters such as SYBER Green I increases the melting temperature (Ririe *et al*, 1997). A control reaction which contained template DNA other than *P. graminea* or no template DNA produced a melting curve with a lower Tm of 83°C was observed which is probably due to the accumulation of primer dimers or non-specific PCR product. This result indicated that the primers were specific for *P. graminea* and could differentiate this pathogen from other closely related *Pyrenophora* spp. and fungal saprophytes isolated from barley seed samples.

### Real-time detection of P. graminea from barley seed

PCR results of screening ten replicate *P. graminea* infected seed samples (Table 1 and Figure 4) indicated that this test was reliably capable of detecting this pathogen from all infection levels constructed by mixing seed lots. Four replicates of the lowest infection level in agar plate tests had 0.5% infection, since there was equal chance of this infection level appearing in four of the replicates tested by PCR, it is likely that the test is capable of detecting a single seed in 200. The test was proved to be robust from the repeatability of results for each of the replicates.

Continuous monitoring of fluorescence emission during rapid cycle PCR (Figure 4) indicated a gradual increase of fluorescence from about cycle 22 due to the accumulation of the specific PCR product from the two infected seed samples. There was increase in fluorescence for the no template control for all the cycles (Figure 4) which is due to the efficient amplification of primer dimers. The accumulation of fluorescence from the no template control was easily distinguished from that of infected seed by the melting curve analysis which was carried out afterwards.

A negative result was obtained from one replicate of a 0.5% infected seed sample and the other was from a replicate of a 5% infected seed sample. Further PCR experiments were carried out on these samples using primers PGF2/PGR2 and ITS 4 and 5. It was demonstrated from these experiments that the 0.5 % sample was a true negative and the 5 % sample was a negative only because DNA extraction from this sample had failed. This indicates the importance of adequate controls at each stage in the seed health test to monitor its progress and verification of the results.

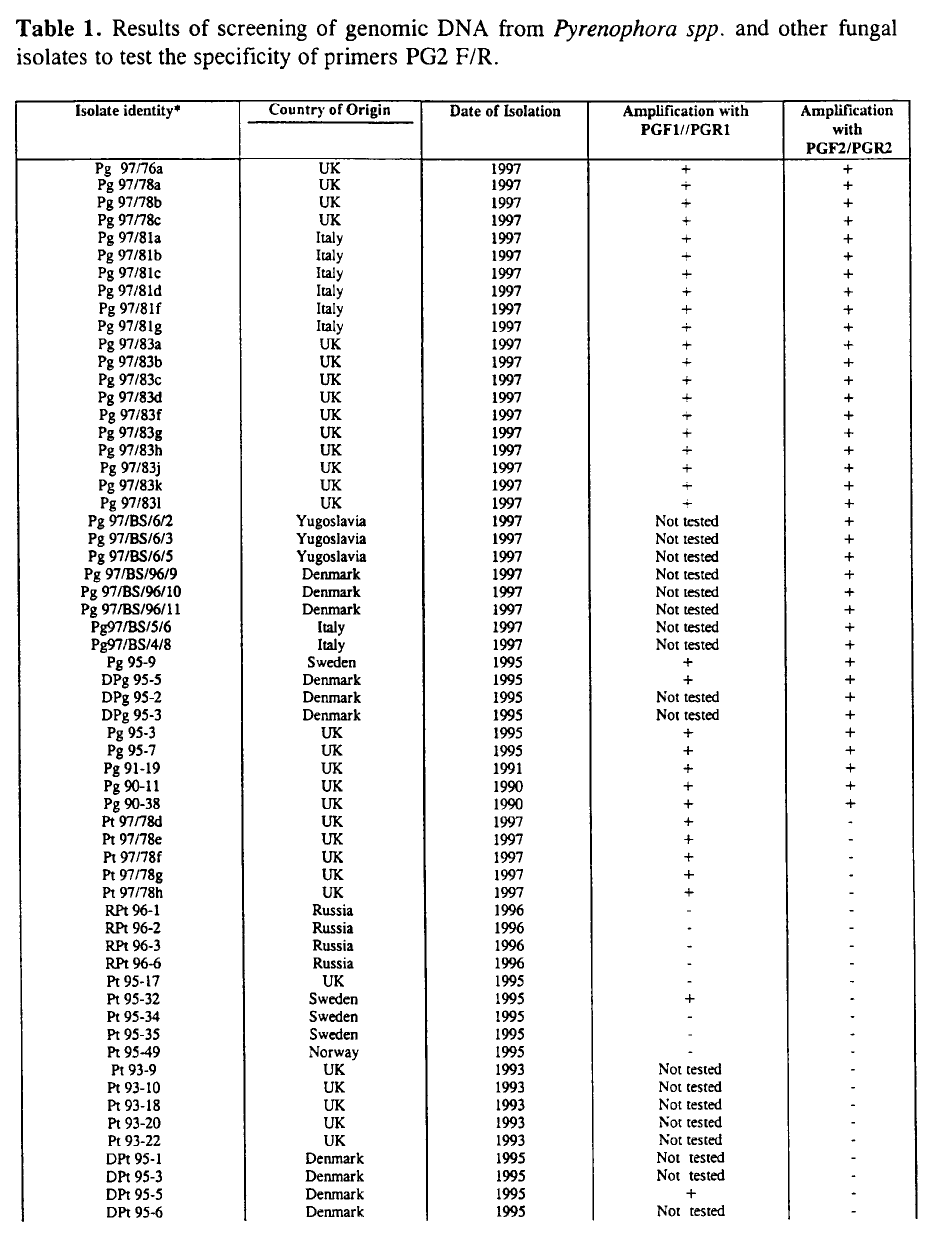

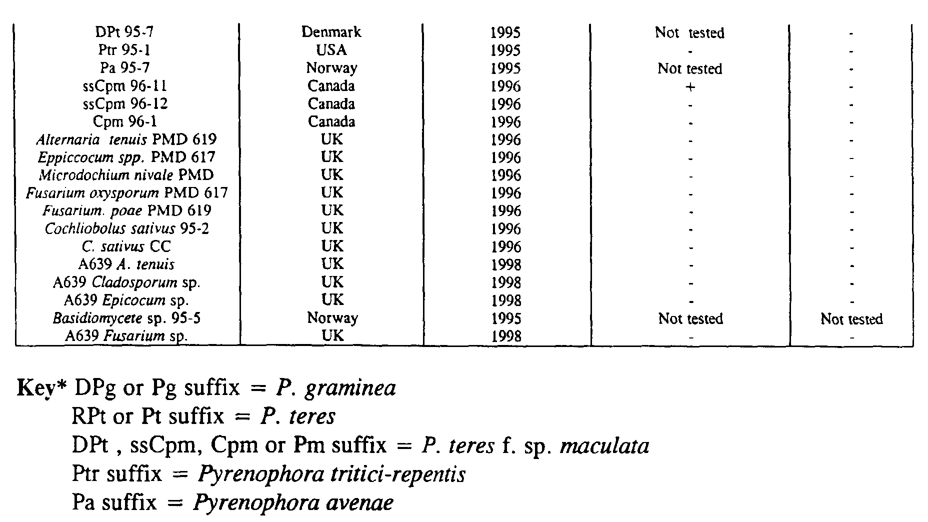

**Table 2**

| Origin of Barley seed used in this study. | | | |
|---|---|---|---|
| **NIAB Code number** | **Variety** | **Origin** | **Percentage *P.*** ***graminae*** **infection (agar Test)** |
| BS2 19/4/96 | Gaellic | NIAB (UK) | 0 |
| 97/76 | Gaulois | UniRoyal Chemical Ltd (UK) | 26.5 |
| 97/79 | Flamenco | UniRoyal Chemical Ltd (UK) | 43 |

**Table 3**

| Comparison of agar test and PCR results for detection of *P.graminea* from barley seed | | | |
|---|---|---|---|
| **Mean % infection level on agar plates** | **Range of % infection on agar plates** | **Total number of positive results with agar plate test** | **Total number of positive results with PCR test.** |
| 1.1 | 0-4.5 | 8 | 9 |
| 3.0 | 0-5 | 9 | 10 |
| 4.4 | 1.5-8.5 | 10 | 10 |
| 6.1 | 2-8.5 | 10 | 10 |
| 9.2 | 3.5-15.0 | 10 | 10 |
| 12.3 | 6-15.5 | 10 | 9* |

| | | | |
|---|---|---|---|
| * One sample failed in the DNA extraction procedure and so produced a negative PCR result. | | | |

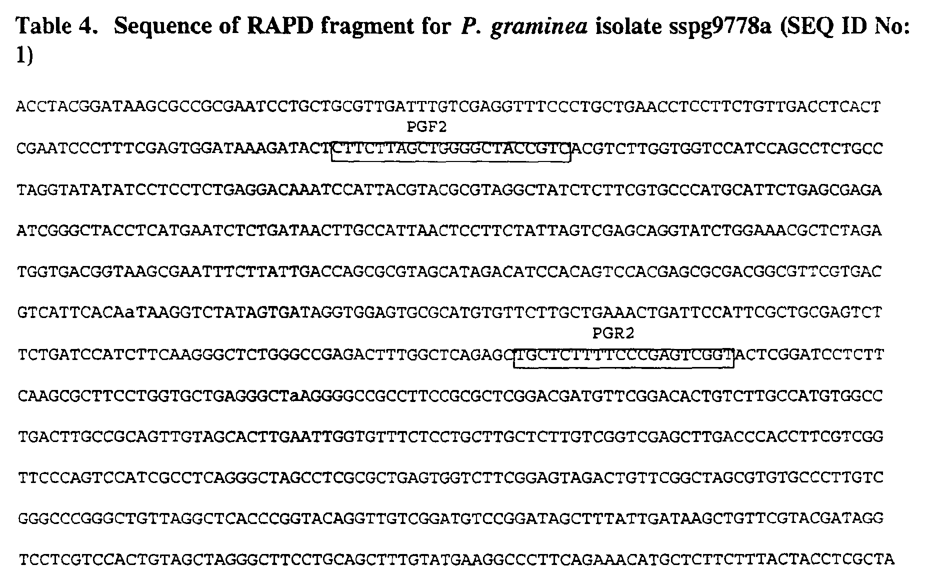

### References

Cockerell V, Rennie WJ and Jacks M 1995. Incidence and control of leaf stripe (*Pyrenophora graminea*) in Scottish barley during the period 1987-1992. Plant Pathology **44**, 655-661.

Cockerell V, Rennie WJ, 1996. Survey of seed-borne pathogens in certified and farm-saved seed in Britain between 1992 and 1994. *Home-Grown Cereals Authority Project report* **124**. London: Home Grown Cereals Authority.

Johanson A, Crowhurst RN, Rikkerink EHA, Fullerton RA and Templeton MD, 1994. The use of species-specific DNA probes for the identification of *Mycosphaerella fijiensis* and *M. musicola*, the causal agents of Sigatoka disease of banana. Plant Pathology **43**, 701-701.

Parry DW and Nicholson P, 1996. Development of a PCR assay to detect *Fusarium poae* in wheat. Plant Pathology, **45**, 383-391.

Platenkamp R, 1976. Investigation of the infection pathway of *Drechslera graminea* in germinating barley. Royal Veterinary and Agricultural University Yearbook 1976, 49-64.

Reeves JC, Ball S, 1991. Preliminary results on the identification of *Pyrenophora* species using DNA polymorphisms amplified from arbitrary primers. Plant varieties and seeds, **4**, 185-189.

Rennie WJ, Tomlin MM, 1984 Barley Leaf Stripe Working sheet number 6. In: *ISTA handbook on Seed health testing*. Zurich, Switzerland: ISTA.

Ririe KM, Rasmussen RP, Wittwer CT, 1997. Product Differentiation by analysis of DNA melting curves during the polymerase chain reaction. Analytical Biochemistry **245**, 154-160.

Sambrook J, Fritsch EF, Maniatis T, 1989. Molecular cloning: a laboratory manual, 2^{nd} edn. USA: Cold Spring Harbour press, 9.38-9.40

Smedegaard-Peterson V, 1983. Cross fertility and genetic relationship between *P.teres* and *P. graminea*. The causes of net blotch and leaf stripe of barley. Seed Science and Technology **11**, 673-680.

Stevens E A, Blakemore E J A,Reeves J C, 1998. Relationships amongst isolates of *Pyrenophora* spp. based on the sequences of the ribosomal DNA spacer regions. Molecular Plant Pathology On-Line, http://www.bspp.org.uk/mppol/1998/1111stevens

Stevens E A, Blakemore E J A and Reeves J C, (1997). Development of a PCR-based test to detect and identify *Pyrenophora* spp. pathogenic on barley. In: *Seed Health Testing:*

*Progress towards the 21*^{*st*} *Century*. J. D. Hutchins and J.C. Reeves (eds). CAB International, Oxon, UK. 139-146.

Stevens EA, Alderson J, Blakemore EJA, Reeves JC 1996. Development of a multiplex PCR seed health test to detect and differentiate three pathogens of barley. In:Marshall G, ed. Diagnostics in Crop Protection Symposium proceedings of British Crop Protection Conference 1996.University of Warwick, UK: no.4, 99-104

Thomas J E, Reeves J C, Thomas E J A, and Kenyon D M (1998) The development of new diagnostic techniques and their role in improving treatment strategies for seed-borne diseases. *Brighton Crop Protection Conference*: Pests and Diseases, **3** 787-792

Welsh J, McClelland M, 1990. Fingerprinting genomes using PCR with arbitrary primers. Nucleic Acid Research **18**, 7213-7218.

White TJ Bruns T, Lee S, Taylor J, 1990. Amplification and direct sequencing of fungal ribosomal RNA genes for phylogenetics. In: Innis MA, Gelfand DH, Sninsky JJ, White TJ, eds. *PCR protocols: A Guide to Methods and Applications*. USA: Academic Press inc., 315-322.

Woo THS, Patel BKC, Smythe LD, Symonds ML, Norris MA, Weyant RS, Dohnt MF, 1998. Identification of *Leptospira inadai* by continuous monitoring of fluorescence during rapid cycle PCR. Systematic and Applied Microbiology **21**, 89-96.

Woo THS, Patel BKC, Smythe LD, Symonds ML, Norris MA and Dohnt, MF, 1997. Identification of pathogenic *Leptospira* genospecies by continuous monitoring of fluorogenic hybridisation probes during rapid-cycle PCR. Journal of Clinical Microbiology **35**, 3140-3146.

Xue B, Goodwin PH, Annis SL 1992. Pathotype identification of *Leptosphaeria maculans* with PCR and oligonucleotide primers from ribosomal internal transcribed spacer sequences. Physiological and Molecular Plant Pathology **41**, 179-188.

## Claims

1. A nucleotide sequence obtainable from *Pyrenophora graminea* comprising the sequence presented SEQ ID No: 1 in Figure 1 and Table 4 or a variant of the sequence.

2. A sequence according to claim 1, comprising any one of the sequences shown in Figure 1.

3. An oligonucleotide comprising at least 10 consecutive bases as occurring in a nucleotide sequence in accordance with claim 1 or 2, or the complement of such a sequence.

4. An oligonucleotide according to claim 3, comprising at least 15, preferably about 20, consecutive bases.

5. An oligonucleotide according to claim 2 or 3, comprising consecutive bases as occurring in SEQ ID No: 1.

6. An oligonucleotide according to claim 3, 4 or 5, comprising a sequence which will hybridise under stringent PCR conditions to a nucleotide sequence present in the genome of only one *Pyrenophora* species.

7. An oligonucleotide according to any one of claims 3 to 6, comprising a sequence which will hybridise under stringent PCR conditions to a nucleotide sequence present in the genome of *P. graminea* but will not hybridise to sequences present in other *Pyrenophora* species, particularly *P. teres.*

8. A pair of oligonucleotides, one comprising at least 10 consecutive bases as occurring in a first portion of a nucleotide sequence in accordance with claim 1 or 2, and the other comprising at least 10 bases complementary to a second portion of a nucleotide sequence in accordance with claim 1 or 2, such that the oligonucleotides are capable of acting as primers in a polymerase chain reaction and will amplify a region of the sequence of only one *Pyrenophora* species, particularly *P. graminea.*

9. A pair of oligonucleotides according to claim 8, wherein each oligonucleotide comprises at least 15 bases, preferably about 20 bases.

10. An oligonucleotide having the sequence 5'CTTCTTAGCTGGGCTACCGTC 3' (SEQ ID No: 2).

11. An oligonucleotide having the sequence 5'ACCGACTCGGGAAAAGAGCA 3' (SEQ ID No: 3).

12. A pair of oligonucleotides comprising the oligonucleotides of claims 10 and 11.

13. An oligonucleotide having the sequence 5'GTTTCTACCTACGGATAGC 3' (SEQ ID No: 4).

14. An oligonucleotide having the sequence 5'TAGCGAGGTAGTAAAGAAGC3' (SEQ ID No: 5).

15. A pair of oligonucleotides, comprising the oligonucleotides of claims 13 and 14.

16. A method of detecting the presence of a specific *Pyrenophora* species, particularly *P. graminea* in plant material, particularly barley seeds, comprising
a) extracting nucleic acid from a sample of plant material;
b) performing PCR using as primers a pair of oligonucleotides in accordance with any one of claims 8, 9, 12 or 15.
c) detecting the presence of any PCR products.

17. A kit for performing a polymerase chain reaction, comprising a pair of oligonucleotides in accordance with any one of claims 8, 9, 12 or 15.


SEQUENCE LISTING

<110> The Minister of Agriculture, Fisheries and Food, i

<120> Improvements in or relating to detection of
      microorganisms in plant material

<130> C326.01N

<140>
<141>

<150> GB 9923022.9
<151> 1999-09-30

<160> 5

<170> PatentIn Ver. 2.1

<210> 1
<211> 961
<212> DNA
<213> Pyrenophora graminea

<400> 1
acctacggat aagcgccgcg aatcctgctg cgttgatttg tcgaggtttc cctgctgaac 60
ctccttctgt tgacctcact cgaatccctt tcgagtggat aaagatactc ttcttagctg 120
gggctaccgt cacgtcttgg tggtccatcc agcctctgcc taggtatata tcctcctctg 180
aggacaaatc cattacgtac gcgtaggcta tctcttcgtg cccatgcatt ctgagcgaga 240
atcgggctac ctcatgaatc tctgataact tgccattaac tccttctatt agtcgagcag 300
gtatctggaa acgctctaga tggtgacggt aagcgaattt cttattgacc agcgcgtagc 360
atagacatcc acagtccacg agcgcgacgg cgttcgtgac gtcattcaca ataaggtcta 420
tagtgatagg tggagtgcgc atgtgttctt gctgaaactg attccattcg ctgcgagtct 480
tctgatccat cttcaagggc tctgggccga gactttggct cagagctgct cttttcccga 540
gtcggtactc ggatcctctt caagcgcttc ctggtgctga gggctaaggg gccgccttcc 600
gcgctcggac gatgttcgga cactgtcttg ccatgtggcc tgacttgccg cagttgtagc 660
acttgaattg gtgtttctcc tgcttgctct tgtcggtcga gcttgaccca ccttcgtcgg 720
ttcccagtcc atcgcctcag ggctagcctc gcgctgagtg gtcttcggag tagactgttc 780
ggctagcgtg tgcccttgtc gggcccgggc tgttaggctc acccggtaca ggttgtcgga 840
tgtccggata gctttattga taagctgttc gtacgatagg tcctcgtcca ctgtagctag 900
ggcttcctgc agctttgtat gaaggccctt cagaaacatg ctcttcttta ctacctcgct 960
a 961

<210> 2
<211> 21
<212> DNA
<213> Pyrenophora graminea

<400> 2
cttcttagct gggctaccgt c                                                21

<210> 3
<211> 20
<212> DNA
<213> Pyrenophora graminea

<400> 3
accgactcgg gaaaagagca                                                  20

<210> 4
<211> 19
<212> DNA
<213> Pyrenophora graminea

<400> 4
gtttctacct acggatagc                                                   19

<210> 5
<211> 20
<212> DNA
<213> Pyrenophora graminea

<400> 5
tagcgaggta gtaaagaagc                                                  20